# EUROPEAN PATENT APPLICATION

(11) **EP 4 270 318 A1**
(43) Date of publication of application: **01.11.2023**
(21) Application number: 22170018.0
(22) Date of filing: 26.04.2022
(51) Int. Cl.: G06T 7/73, G06T 7/00

(54) **METHOD AND SYSTEM FOR X-RAY IMAGE QUALITY ASSURANCE**

(71) Applicant: Koninklijke Philips N.V., 5656 AG Eindhoven (NL)
(72) Inventor: SCHULZ, Heinrich, Eindhoven (NL); SAALBACH, Axel, Eindhoven (NL); SIEREN, Malte, Eindhoven (NL); BARKHAUSEN, Jörg, Eindhoven (NL); HANSEN, Lasse, Eindhoven (NL); HOBE, Malte, Eindhoven (NL); Heinrich, Mattias, Eindhoven (NL)
(74) Representative: Philips Intellectual Property & Standards

(57) **Abstract**

The invention provides a computer-implemented method for X-Ray image quality assurance. The method comprises acquiring X-Ray image data (S1) of a subject using a mobile X-Ray imaging system, the X-Ray image data comprising at least one X-Ray image, based on the X-Ray image data, performing an automatic image-based detection of imaging errors indicative of a misalignment of the X-Ray imaging system relative to the subject (S2), and in response to detecting an imaging error indicative of a misalignment, prompting a display device to display a user interface comprising an indicator indicating a presence of the misalignment and/or an indicator indicating a type of the misalignment and/or an indicator indicating a degree of the misalignment (S3).

## Description

### FIELD OF THE INVENTION

The invention provides a method and system for X-Ray image quality assurance, a computer program product, and a computer readable medium.

### BACKGROUND OF THE INVENTION

X-Ray imaging is the modality of choice when it comes to the verification of line and tube positioning in the Intensive Care Unit (ICU) and the Emergency Department (ED). This assessment is typically performed based on the location of a device (e.g. in terms of the device tip), and its relation to specific anatomical landmarks or regions. Moreover, it is usually one of the first modalities to address and follow-up thoracic pathologies.

Due to challenging imaging conditions in ICU and ED, mobile chest radiography is susceptible to a broad range of imaging problems. In this context, especially projection errors and distortions, resulting from a sub-optimal positioning of the patient and/or configuration of the X-Ray system, could result in erroneous measurements and potentially wrong diagnosis.

It is assumed that mobile chest radiography contributes to a large share of all chest X-Ray images. In ICU and ED settings, mobile imaging is commonly employed to verify the correct positioning of lines and tubes such as Central Venous Catheters (CVCs) or Endotracheal Tubes (ETT). Erroneous positioning of these devices can result in severe adverse events, such as a pneumothorax, with potential life-threatening consequences.

Therefore, daily confirmations via X-Ray imaging are frequently employed. As an example, for an assessment of the ETT position, typically the distance between the tip of the ETT and the carina bifurcation is evaluated, a measurement which can be severely affected by projection errors and distortions. Moreover, as mentioned above, such imaging is usually one of the first modalities to address and follow-up thoracic pathologies. These pathologies may be obscured or misinterpreted if the radiographs do not conform to the standardized projections.

Mobile X-Ray imaging encounters challenges due to poor patient conditions, the ICU/ED room situation, and/or the involved equipment. Thus, there may be quality issues due over-exposure or under-exposure, but also quality issues due patient positioning or rotation relative to the imaging system, which is a common occurrence in X-Ray imaging, particularly mobile imaging.

Ideally, the median coronal plane of the detector should be parallel to the chest and perpendicular to the central ray of the X-Ray system. In the above-described conditions, this setup often difficult to achieve.

Deviations can result in cephalic or caudal angulations which do not only affect the appearance of the anatomy in the image but could influence the reliability of measurements, AI results, and the assessment of pathologies. Angulations are amongst the most frequent quality problems in X-Ray imaging.

As can be seen from the above, ideal X-Ray imaging conditions are sometimes difficult or impossible to attain, particularly for mobile X-Ray imaging, for example in the ICU or ED room. Consequently, image quality may be lacking, and patients may be put at risk.

### SUMMARY OF THE INVENTION

It is an object of the present invention to allow for reducing the negative effects of poor image quality and potential risk for a patient.

The invention provides a computer-implemented method for X-Ray image quality assurance. The method comprises acquiring X-Ray image data of a subject using a mobile X-Ray imaging system, the X-Ray image data comprising at least one X-Ray image, based on the X-Ray image data, performing an automatic image-based detection of imaging errors indicative of a misalignment of the X-Ray imaging system relative to the subject, and in response to detecting an imaging error indicative of a misalignment, prompting a display device to display a user interface comprising an indicator indicating a presence of the misalignment and/or an indicator indicating a type of the misalignment and/or an indicator indicating a degree of the misalignment.

Thus, the negative effects of poor X-Ray image quality can be mitigated by detecting such quality issues that are particularly relevant, for example for mobile chest X-Ray imaging, and alerting medical personnel, ensuring appropriate use of the X-Ray images and ensuring that appropriate actions can be initiated.

As can be seen from the above, the method relates to an image-based detection of projections errors, e.g. resulting from the patient positioning, or distortions, e.g. resulting from an increased distance between the source and the patient, in X-Ray acquisitions. To this end, different techniques, described in more detail below, may be employed, including, for example, AI methods for image classification and/or regression, as well as based on segmentation and landmark detection. The method may also provide a user-interface which alerts a user, e.g., radiologists and ICU/ED staff, about quality issues, like the presence and/or degree of angulations and/or misalignment of a patient and an X-Ray system, thereby allowing for appropriate action and/or interpretation of the X-Ray images.
The method of the present disclosure can be used in an X-Ray imaging system, e.g., for an early detection of misplaced devices, as well as part of a PACS viewer or a diagnostic workstation. Alternatively or in addition, the method can be used to inform radiologists and ICU/ED staff about quality issues with X-Ray images that could affect the diagnostic evaluation, i.e., as a result of distortions and/or angulations. This allows for the radiologist or staff to take action, e.g., disregard the images and/or trigger or perform image correction and/or acquiring new images.

The subject may be a person and is also referred to as patient in the present disclosure.

Imaging errors manifest themselves in different aspects of an X-Ray image. As an example, signs of angulations may include the relation of the manubrium with regard to the fourth thoracic vertebra (T4), the position of the clavicles in relation to the spine and the shapes of the ribs.

According to the present disclosure, performing the automatic image-based detection of imaging errors may comprise making use of a Deep Learning method, in particular, making use of a first Deep Learning method for image-classification-based detection of misalignments and/or a second Deep Learning method for landmark-detection-based detection of misalignments.

This allows for a reliable detection while also being non-invasive and do not require concurrently using additional detection means for monitoring the subject. Particularly, the use of radio markers can be avoided.

In view of a wide range of available data on anatomy and a relatively well-defined problem, Deep Learning Methods are well-suited and capable of automatic image-based detection of imaging errors. In case of an image-classification-based detection, as an example, the deep learning method may involve learning based on real and/or artificially created X-Ray images labeled with regard to the presence and/or type and/or degree of misalignments. X-Ray images may then be classified or identified as comprising a misalignment and optionally a misalignment of a specific type or degree.

In case of a landmark-detection-based detection of misalignments, Deep Learning Methods may be used to recognize pre-determined landmarks in an X-Ray image and, based on available anatomy-data, it may be determined automatically that the landmarks have relative positions in the X-Ray image that deviate from expected positions and this may indicate a misalignment. More details in this regard will be provided below.

According to the present disclosure, performing the automatic image-based detection of imaging errors may comprise image-classification-based detection of misalignments making use of a first neural network trained with real and/or artificially created X-Ray images labeled with regard to the presence and/or type and/or degree of misalignments. The first neural network may, for example, be a Convolutional Neural Network, in particular DenseNet or ResNet. Other methods of machine learning are, however, conceivable. The Convolutional Neural Network may be a Deep Convolutional Neural Network.

The real X-Ray images comprised in training data for training the first neural network may be X-Ray images obtained at multiple different and known alignments or misalignments of a subject relative to one or more components of the X-Ray system. Artificial X-Ray images that may be used and how they may be used will be described in more detail further below.

An advantage of classification-based detection is that it is robust and fast while, for many applications, providing the required amount of precision. Particularly, where no precise measurements in X-Ray images are required, precision-requirements are not particularly high.

An example for classification would be a good/medium/bad statement on the image quality, which could thus be an indication for an image retake, which may, e.g. be output to the technician.

According to the present disclosure, the image-classification-based detection may comprise the first neural network performing image classification and/or regression on the X-Ray image data of the subject to detect the imaging error, and in particular to identify the presence and/or type and/or degree of misalignments.

The image classification and/or regression may be performed so as to determine a misalignment, and in particular type and/or degree of misalignment, causing one or more imaging errors that most closely resemble one or more imaging errors in the X-Ray image.

The image classification and regression may be used individually or in combination.

As mentioned above, an example for classification would be a good/medium/bad statement on the image quality. Regression could return a continuous score for the quantity (degrees) of rotation, for example.

Methods known in the art may be used for image classification and/or regression.

According to the present disclosure, performing the automatic image-based detection of imaging errors may comprise landmark-detection-based detection of imaging errors making use of a second neural network configured to detect pre-determined landmarks in an X-Ray image, the landmarks suitable for deriving an imaging error from their arrangement in the X-Ray image, in particular, wherein the second neural network is a Convolutional Neural Network for image segmentation, for example a UNet. Other methods of machine learning are, however, conceivable.

Landmarks, in the present disclosure, may be any anatomical structure detectable by X-Ray. For example, landmarks may be parts of the surface of an anatomical structure. They may be determined, for example, based on surface profile and/or shape of the depicted anatomical structure. Anatomical maps and/or meshes modelling the surface of the anatomical feature may be used in this context.

Based on landmark-positions in the X-Ray image and expected land-mark position, an imaging error may be derived. Landmark-based detection allows for very precise determination of type and/degree of misalignments and may also allow for correcting quantitative measurements, e.g., of distances and/or angles, made in the X-Ray image.

According to the present disclosure, the landmark-detection-based detection of imaging errors may comprise detecting the pre-determined landmarks in the X-Ray image by means of the second neural network, determining an arrangement of, in particular a distance between, the detected landmarks in the X-Ray image, and, based thereon, detecting the imaging error, and in particular the presence and/or type and/or degree of misalignments.

For example, it may be determined that a misalignment is present when the arrangement of the landmarks in the X-Ray image deviates from an expected arrangement. Moreover, the arrangement may be used to determine the degree of a misalignment and, based thereon, optionally corrected distances, corrected for the imaging error due to the misalignment may be determined.

The predetermined landmarks may be landmarks that have a fixed relative position, e.g., will not move significantly with respect to each other. Their arrangement, particularly distance, may thus be particularly reliable indicator for imaging errors.

According to the present disclosure, detecting an imaging error indicative of a misalignment may be performed using artificially created X-Ray images. In particular, the artificial X-Ray images may be images obtained by applying a plurality of different parametrizations to Digitally Reconstructed Radiographs reconstructed from 3D medical image data, e.g., CT image data, so as to obtain a plurality of artificial X-Ray images with imaging errors representative of different misalignments.

For example, Digitally Reconstructed Radiographs (DRRs) may be obtained from the CT image data to generate artificial X-Rays. Using the artificial X-Rays, by means of different parametrizations of the DRRs, artificial X-Ray images with exact information about the misalignment, e.g., level of angulation and/or distance, can be created.

An advantage of using the artificially created X-Ray images in the manner described above is that it allows for creating a very large and comprehensive set of data, e.g., as training data for machine learning. Higher precision may also be attainable considering that real X-Ray image data may have a wide variety of imaging errors and that not all of these imaging errors may have been accurately identified and labelled in the data set.

According to the present disclosure, the first neural network and/or second neural network may be trained based on the artificially created X-Ray images. In particular, the first neural network may be trained at least with the artificially created X-Ray images labeled with regard to the presence and/or type and/or degree of misalignments.

The use of artificial X-Ray images may allow for a large, comprehensive, well-structured training data set that may comprise different types alone or in combination and different degrees of misalignments. Accordingly, a reliable detection and identification of misalignments can be provided. This may be particularly suitable for a classification algorithm.

According to the present disclosure, detecting an imaging error indicative of a misalignment using artificially created X-Ray images may comprise using the artificially created X-Ray images for automatically determining corrected landmarks.

The artificially created X-Ray images may be X-Ray images as described above, yet in addition or alternatively to being labelled as having a misalignment, the artificially created X-Ray images may, in this case, also comprise labelled landmarks. Based thereon, the landmarks detected in the X-Ray image may be corrected, e.g., by using a machine learning method trained using the labelled artificially created X-Ray images as training data. The labelled artificially created X-Ray images may also be used for identifying the landmarks in the X-Ray image, e.g., by using a machine learning method trained using the labelled artificially created X-Ray images as training data.

Thus, in addition to detecting misalignment, it may also be possible to correct data to some degree. As an example, the corrected landmarks may be displayed on a display device overlaid with the X-Ray image. Optionally, the detected (non-corrected) landmarks may also be displayed on the display device. The distance by which the respective position of the landmark has been corrected may optionally also be displayed on the display device.

An advantage is that correct landmark positions may in many use cases be sufficient, such that it is not necessary to perform more complex, time-consuming, and/or resource intensive methods.

According to the present disclosure, detecting an imaging error indicative of a misalignment using artificially created X-Ray images may comprise using the artificially created X-Ray images for automatically determining a misalignment-induced error or uncertainty margin for measurements made based on the X-Ray image.

For example, where distances between points on the X-Ray image, e.g., landmarks depicted therein, and/or relative orientations are measured in the X-Ray image, depending on the degree and type of misalignment, the actual distance and/or orientation may be different. The error may quantify the deviation of the measured values from expected actual values. The error may be corrected and/or taken into account Where an exact error is not known or not known at a desired accuracy, an uncertainty margin may be provided that represents an estimated limit to the precision of the measurements in the X-Ray image due to the misalignment. Thus, it can be determined, for example, whether and to what degree the X-Ray image is suitable for quantitative assessments.

According to the present disclosure, detecting an imaging error indicative of a misalignment using artificially created X-Ray images may comprise using the artificially created X-Ray images for automatically determining a corrected measurement by correcting misalignment-induced errors of a measurement made based on the X-Ray image

The artificially created X-Ray images may be the artificially created X-Ray images described above and advantages and features described in that context similarly apply in this context. Using them for correcting misalignment-induced errors of measurement may be particularly advantageous, as the effect of a misalignment can be well recognized and isolated from other misalignments and/or other imaging errors, such that the correction will yield results of high precision.

The method of the present disclosure may comprise determining the location of the pre-determined landmarks in the 3D medical image data and in one or more of the artificially created X-Ray images and, based thereon detecting an imaging error and/or determining the corrected landmarks and/or the misalignment-induced error or uncertainty margin and/or corrected measurement.

This method allows for leveraging the above-described general advantages of the artificially created X-Ray images. In addition, 3D medical image data is particularly suitable for using landmark-based methods, as, unlike the X-Ray images, the landmarks can be determined using 3D data, e.g., surface data of an anatomical structure, and their position may be determined in 3D coordinates. This allows for a high-quality prediction of expected landmark positions for a given type and/or degree of misalignment. Accordingly, high precision can be ensured.

The method of the present disclosure may comprise training a/the first neural network with real and/or artificially created X-Ray images labeled with regard to the presence and/or type and/or degree of misalignments.

The features and advantages outlined above in the context of the first neural network and, particularly, in the context of using labeled X-Ray images (real and/or artificial) similarly apply in this context.

The method of the present disclosure may comprise training a/the second neural network to detect pre-determined landmarks in an X-Ray image, in particular by means of image segmentation.

The features and advantages outlined above in the context of the second neural network and, particularly, in the context of detecting and using pre-determined landmarks similarly apply in this context.

The method of the present disclosure may comprise obtaining a/the plurality of artificial X-Ray images, in particular, applying a plurality of different parametrizations to Digitally Reconstructed Radiographs reconstructed from 3D medical image data, e.g., CT image data, so as to obtain a/the plurality of artificial X-Ray images with imaging errors representative of different misalignments.

The features and advantages outlined above in the context of the artificial X-Ray images similarly apply in this context.

According to the present disclosure, the misalignment may comprise an angulation and/or a distance deviation from a target alignment of the X-Ray system relative to the subject. In other words, there may be a deviation from a relative target orientation, e.g. expressed by a deviation from one or more target angles, between subject and one or more components of the X-Ray. Alternatively or in addition, there may be deviations from a target distance between the subject and one or more components of the X-Ray system. These types of misalignments lead to imaging errors that are particularly relevant and can be well-addressed by the present disclosure, i.e., projection errors and distortions.

According to the present disclosure, the imaging error may comprise a projection error and/or a distortion. A projection error may be brought about, for example, by non-ideal relative orientation, e.g., an angulation, of the subject and the X-Ray system during X-Ray acquisition. A distortion may be brought about, for example, by a deviation from an ideal distance, i.e., a distance deviation, between the X-Ray source and subject during X-Ray acquisition.

According to the present disclosure, the X-Ray image may depict a chest of the subject. In particular, the X-Ray image may depict at least one of the the sternum, particularly the manubrium, vertebrae, in particular at least one of the thoracic vertebrae, e.g., the fourth thoracic vertebra (T4), the clavicle and one or more ribs. Chest X-Rays are often obtained by mobile X-Ray applications, e.g., for ICU or ED settings. These types of X-Ray images require particular care and quality control, as low quality may have significant adverse effects, compared to, for example, simple diagnostic imaging to detect a broken limb. Therefore, the claimed method will be of particular relevance in this context.

According to the present disclosure, the subject may be an immobile person, in particular, a person in a lying position. Accordingly, it may be necessary to arrange the X-Ray system in a non-ideal orientation to accommodate for the immobile person not being able to be brought into a position that would allow for a better orientation of the X-Ray system. This increases the risk of quality issues and, therefore, the claimed method will be of particular relevance in this context.

According to the present disclosure, the method is performed on a subject in an ICU and/or ED setting. Subjects in an intensive care unit, ICU, or an emergency department, ED, setting are often immobile and/or difficult to position, particularly as they may have monitoring and/or treatment machines attached to them. Moreover, their condition may not be robust. Accordingly, it may be necessary to arrange the X-Ray system in a non-ideal orientation to accommodate for the subject in the ICU or ED setting. This increases the risk of quality issues and, therefore, the claimed method will be of particular relevance in these settings.

The invention also provides a system for X-Ray quality assurance. The system comprises a processing system configured to carry out and/or control any of the method steps described in the present disclosure, in particular, carry out the computing or data processing steps of the present disclosure and/or control data acquisition, e.g., by controlling an/the X-Ray imaging system to acquire the X-Ray image data and/or a/the CT imaging system to acquire CT image data, and/or to control a display device to display the user interface.

The system according to the present disclosure may comprise a mobile X-Ray imaging system configured to acquire the X-Ray image data, and optionally to provide the X-Ray image data to the processing system.

Alternatively of in addition, the system according to the present disclosure may comprise a display device configured to display the user interface.

Alternatively or in addition, the system according to the present disclosure may comprise a CT imaging system for obtaining the 3D medical image data.

Alternatively or in addition, the system according to the present disclosure may comprise one or more instruments, in particular, instruments to be inserted into the subject and depicted in the X-Ray image. For example, instruments may include a tube and/or a probe.

The invention also provides a computer program product comprising instructions which, when the program is executed by a computer, cause the computer to carry out and/or control any of the method steps described in the present disclosure.

The invention also provides a computer readable medium comprising instructions which, when executed by a computer, cause the computer to carry out and/or control any of the method steps described in the present disclosure.

The features and advantages outlined in the context of the method for quality assurance similarly apply to the system, the computer program product, and the computer readable medium of the present disclosure.

Further features, examples, and advantages will become apparent from the detailed description making reference to the accompanying drawings.

### BRIEF DESCRIPTION OF THE DRAWINGS

Fig. 1 shows a schematic illustration of a system according to the present disclosure;
Fig. 2 shows a flow diagram representative of a method according to the present disclosure; and
Figs. 3a to 3c illustrate detection techniques.

### DETAILED DESCRIPTION OF EMBODIMENTS

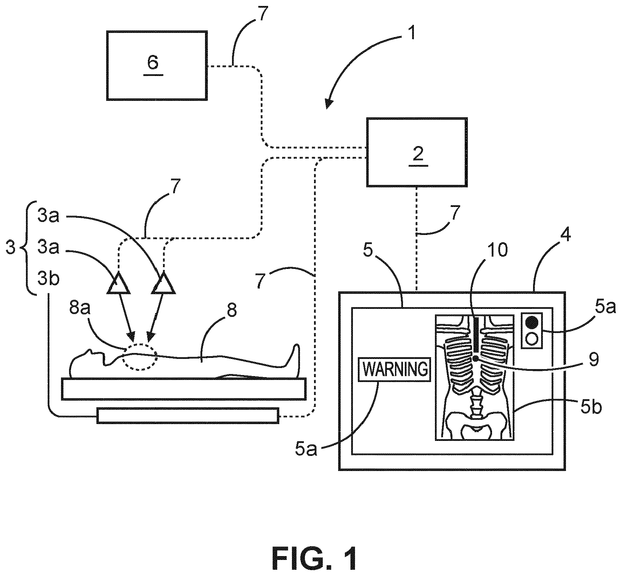

Fig. 1 illustrates a system 1 according to the present disclosure. The system comprises a data processing system 2 and an X-Ray imaging system 3 having X-Ray sources 3a and an X-Ray detector 3b.

The system further comprises a display device 4. The display device is configured to display a user interface 5. The user interface 5 in the present case outputs an indicator 5a and an X-Ray image 5b.

The system may optionally comprise a CT imaging system 6 and data connections 7 connecting one or more of the other elements of the system.

Fig. 1 also shows, for the sake of illustration, a subject, e.g., a patient, 8 whose chest 8a is shown in the X-Ray image. The subject is shown in a lying-down position and may, for example, be in an ICU setting.

The X-Ray sources, in this example, are arranged over the subject and the X-Ray detectors are arranged below the subject.

Fig. 1 shows an example wherein, in the X-Ray image, a landmark 9 and an optional instrument 10, which need not be part of the system, are depicted.

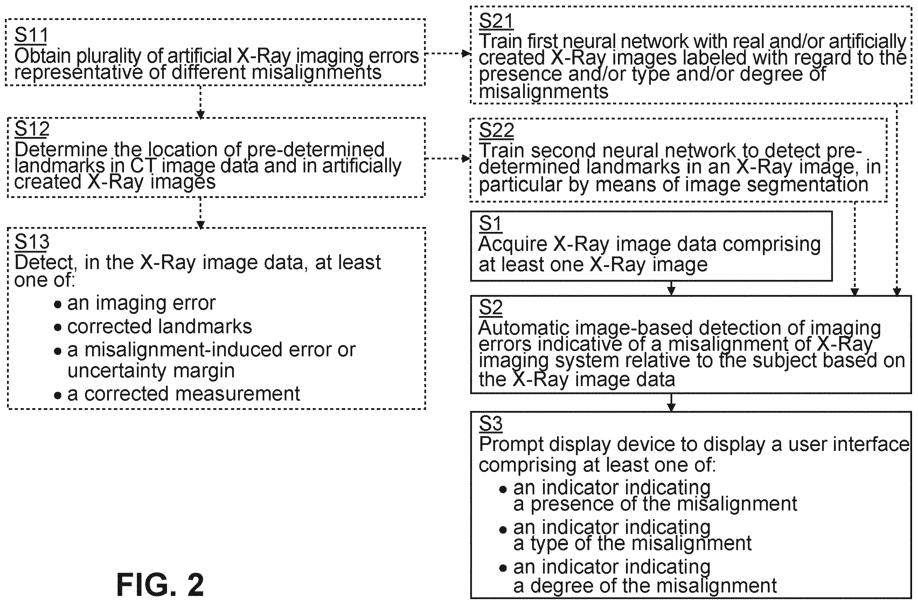

In the following, making reference to Fig. 2, a computer-implemented method according to the present disclosure is described, which may, for example, be carried out by a system according to the present disclosure, in particular, a system as described above and shown in Fig. 1 or as claimed.

In step S1, X-Ray image data of a subject is acquired using a mobile X-Ray imaging system, the X-Ray image data comprising at least one X-Ray image.

In step S2, based on the X-Ray image data, an automatic image-based detection of imaging errors indicative of a misalignment of the X-Ray imaging system relative to the subject is performed.

In step S3, in response to detecting an imaging error indicative of a misalignment, a display device is prompted to display a user interface comprising an indicator indicating a presence of the misalignment and/or an indicator indicating a type of the misalignment and/or an indicator indicating a degree of the misalignment.

Optional steps S11 and S12 may be performed prior to, during, or after step S1.

In optional step S11, a plurality of artificial X-Ray images with imaging errors representative of different misalignments are obtained is obtained, in this example by applying a plurality of different parametrizations to Digitally Reconstructed Radiographs reconstructed from CT image data.

In optional step S12, subsequently to step S11, the location of pre-determined landmarks is determined in the CT image data and in one or more of the artificially created X-Ray images.

Based thereon, in optional step S13, an imaging error and/or the corrected landmarks and/or the misalignment-induced error or uncertainty margin and/or corrected measurement are detected in the X-Ray image data.

In optional step S13, promoting a display device to display a user interface comprising a visual representation of at least one of the detected imaging error and/or corrected landmarks and/or misalignment-induced error or uncertainty margin and/or corrected measurement are detected in the X-Ray image data. The user interface in this step may be the user interface of step S3.

In optional step S21, a first neural network is trained with real and/or artificially created X-Ray images labeled with regard to the presence and/or type and/or degree of misalignments. The artificial image data may be those obtained in step S11. In optional step S22, which may be performed alternatively or in addition to step S21, a second neural network is trained to detect pre-determined landmarks in an X-Ray image, in particular by means of image segmentation. The landmarks obtained in step S12 may be used for training the second neural network. Steps S21 and S22 may be performed prior to the automatic image-based detection of imaging errors. Their respective output may be used as input for the automatic image-based detection of imaging errors.

According to the present disclosure, an image classification/regression-based method may be employed for detecting imaging errors. That is, the identification of imaging errors may be formulated as an image classification or regression problem. The problem can be addressed for example using Deep Learning methods, and Deep Convolutional Neural Networks in particular.

For example, given a dataset of X-Ray images which are labeled, e.g., with regard to the presence of angulations, a network (e.g., DenseNet, ResNet etc.) can be trained to discriminate between categories, e.g., normal alignment and different categories of misalignments, particularly angulations. Additionally or alternatively, labels or annotations indicating the severity of the misalignment, particularly angulation, e.g., as a continuous score may be provided. The network may then be trained to predict the presence, type, and optionally degree of the imaging error, e.g., a projection error. The classification/regression approach may be seen as providing an end-to-end solution for the detection of imaging errors and it could be considered as a black box approach.

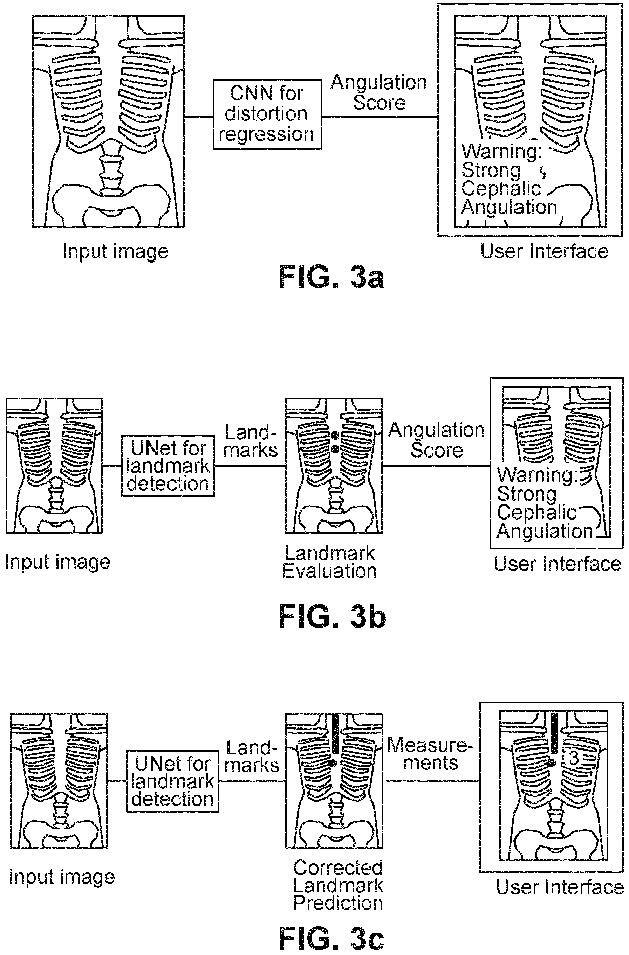

Examples are shown in Figs. 3a to 3c. Fig. 3a illustrates a regression algorithm to automatically determine the amount of rotation. Fig. 3b illustrates automatic landmark detection algorithm for rule-based angulation assessment. Fig. 3c illustrates landmark detection for prediction of adjusted position based on angulation correction.

Fig. 3a illustrates, merely as an example, an angulation score is determined, and a warning is output stating that there is a strong cephalic angulation. However, instead, only the presence of an angulation may be detected, and the user interface may not indicate the type and degree of angulation. Various other combinations are conceivable. Although Fig. 3a refers to a CNN for distortion regression, this need not necessarily be used.

In other examples, according to the present disclosure, landmark-based detection may be employed. For example, to reflect the assessment of an image by a radiologist, landmarks-based approaches are applied. Using Deep Learning methods for landmark detection / segmentation (e.g., UNets, as exemplarily shown in Fig. 3), structures like the clavicles, the manubrium and T4 can be located in the image. The (particularly signed) distance between structures in the image gives a direct indication about the degree of the projection error. Accordingly, based on landmark evaluation, as shown in Fig. 3b, a warning that an imaging error is present and, optionally the type and degree thereof, in this case a strong cephalic angulation, may be displayed as part of the user interface.

Since image data and corresponding annotations are often difficult to obtain, data simulation strategies may be employed according to the present disclosure. For example, using Computer Tomography (CT) images of patients, Digitally Reconstructed Radiographs (DRRs) can be used to generate artificial X-Rays. Using the artificial X-Rays, by means of different parametrizations of the DRRs, an arbitrary number of artificial X-Ray images with exact information about the level of angulation / distance can be created. Based thereon, networks, e.g., the above-described networks may be trained to detect presence, type, and/or degree if an imaging error.

In addition to the location of landmarks in the artificial X-Ray images, the location of the landmarks in the CT may be available, and hence, exact measurement may be possible. An example is shown in Fig. 3c. As an example, a network, e.g., the UNet, is used for landmark detection and, corrected landmarks may be predicted and optionally an absolute value for distances in the image may be provided, in particular, based also on the landmarks in the CT image data.

According to the present disclosure, regression techniques could be used to predict the accurate measurements from the image directly and/or to predict an uncertainty margin (with regard to the image-based measurements) and/or segmentation techniques could be used to predict projection error corrected landmarks which allow for an exact measurement.

Based on the outcome of the previously described techniques/algorithms, visual feedback can be given to a user of the system, as already described above. For example, depending on the type of the outcome, this can take the form of a binary indicator (e.g., "red-light" / "green-light"), suggesting that the image is either suitable for assessment or not. Alternatively or in addition, an indicator having more than two values, e.g., a continuous indicator, could be displayed to the user.

While the invention has been illustrated and described in detail in the drawings and foregoing description, such illustration and description are to be considered exemplary and not restrictive. The invention is not limited to the disclosed embodiments. In view of the foregoing description and drawings it will be evident to a person skilled in the art that various modifications may be made within the scope of the invention, as defined by the claims.

**LIST OF REFERENCE SIGNS:**

| System 1 | |
|---|---|
| Data processing system | 2 |
| X-Ray imaging system | 3 |
| X-Ray source | 3a |
| X-Ray detector | 3b |
| Display device | 4 |
| User interface | 5 |
| Indicator | 5a |
| Image | 5b |
| CT imaging system | 6 |
| Data connections | 7 |
| Patient | 8 |
| Chest | 8a |
| Landmark | 9 |
| Instrument | 10 |

## Claims

1. A computer-implemented method for X-Ray quality assurance, the method comprising
acquiring (S1) X-Ray image data of a subject (8) using a mobile X-Ray imaging system (3), the X-Ray image data comprising at least one X-Ray image;
based on the X-Ray image data, performing (S2) an automatic image-based detection of imaging errors indicative of a misalignment of the X-Ray imaging system (3) relative to the subject (8); and
in response to detecting an imaging error indicative of a misalignment, prompting (S3) a display device (4) to display a user interface (5) comprising an indicator indicating a presence of the misalignment and/or an indicator indicating a type of the misalignment and/or an indicator indicating a degree of the misalignment.

2. The method of claim 1, wherein performing the automatic image-based detection of imaging errors comprises making use of a Deep Learning method, in particular, making use of a first Deep Learning method for image-classification-based detection of misalignments and/or a second Deep Learning method for landmark-detection-based detection of misalignments.

3. The method of claim 1 or claim 2, wherein performing the automatic image-based detection of imaging errors comprises image-classification-based detection of misalignments making use of a first neural network trained with real and/or artificially created X-Ray images labeled with regard to the presence and/or type and/or degree of misalignments, in particular, wherein first neural network is a Convolutional Neural Network.

4. The method of claim 3, wherein the image-classification-based detection comprises the first neural network performing image classification and/or regression on the X-Ray image data of the subject to detect the imaging error, and in particular to identify the presence and/or type and/or degree of misalignments.

5. The method of any of the preceding claims, wherein performing the automatic image-based detection of imaging errors comprises landmark-detection-based detection of imaging errors making use of a second neural network configured to detect pre-determined landmarks (9) in an X-Ray image, the landmarks suitable for deriving an imaging error from their arrangement in the X-Ray image, in particular, wherein the second neural network is a Convolutional Neural Network for image segmentation.

6. The method of claim 5, wherein the landmark-detection-based detection of imaging errors comprises detecting the pre-determined landmarks (9) in the X-Ray image by means of the second neural network, determining an arrangement of, in particular a distance between, the detected landmarks, and, based thereon, detecting the imaging error, and in particular the presence and/or type and/or degree of misalignments.

7. The method of any of the preceding claims,
wherein detecting an imaging error indicative of a misalignment is performed using artificially created X-Ray images,
in particular, wherein the artificial X-Ray images are images obtained by applying a plurality of different parametrizations to Digitally Reconstructed Radiographs reconstructed from 3D medical image data, e.g., CT image data, so as to obtain a plurality of artificial X-Ray images with imaging errors representative of different misalignments.

8. The method of claim 7,
wherein the first neural network and/or second neural network is trained based on the artificially created X-Ray images, in particular, wherein the first neural network is trained at least with the artificially created X-Ray images labeled with regard to the presence and/or type and/or degree of misalignments; and/or
wherein detecting an imaging error indicative of a misalignment using artificially created X-Ray images comprises using the artificially created X-Ray images for automatically determining corrected landmarks; and/or
wherein detecting an imaging error indicative of a misalignment using artificially created X-Ray images comprises using the artificially created X-Ray images for automatically determining a misalignment-induced error or uncertainty margin for measurements made based on the X-Ray image; and/or
wherein detecting an imaging error indicative of a misalignment using artificially created X-Ray images comprises using the artificially created X-Ray images for automatically determining a corrected measurement by correcting misalignment-induced errors of a measurement made based on the X-Ray image.

9. The method of claim 7 or claim 8, wherein the method comprises determining (S12) the location of the pre-determined landmarks in the 3D medical image data and in one or more of the artificially created X-Ray images and, based thereon, detecting (S13) an imaging error and/or determining the corrected landmarks and/or the misalignment-induced error or uncertainty margin and/or corrected measurement.

10. The method of any of the preceding claims, comprising
training (S21) a/the first neural network with real and/or artificially created X-Ray images labeled with regard to the presence and/or type and/or degree of misalignments; and/or
training (S22) a/the second neural network to detect pre-determined landmarks in an X-Ray image, in particular by means of image segmentation; and/or
obtaining (S11) a/the plurality of artificial X-Ray images, in particular, applying a plurality of different parametrizations to Digitally Reconstructed Radiographs reconstructed from 3D medical image data, e.g., CT image data, so as to obtain a/the plurality of artificial X-Ray images with imaging errors representative of different misalignments.

11. The method of any of the preceding claims,
wherein the misalignment comprises an angulation and/or a distance deviation from a target alignment of the X-Ray system (3) relative to the subject (8), and/or
wherein the imaging error comprises a projection error and/or a distortion, and/or
wherein the X-Ray image depicts a chest (8a) of the subject (8), and/or
wherein the subject (8) is an immobile person, in particular, a person in a lying position, and/or
wherein the method is performed on a subject (8) in an ICU and/or ED setting.

12. A system (1) for X-Ray quality assurance, the system comprising
a processing system (2) configured to carry out and/or control the steps of the method of any of claims 1 to 11,
the system (1) in particular comprising
a mobile X-Ray imaging system (3) configured to acquire the X-Ray image data, and optionally to provide the X-Ray image data to the processing system; and/or
a display device (4) configured to display the user interface (5); and/or
a CT imaging system (6) for obtaining the 3D medical image data; and/or
one or more instruments (10), in particular, instruments to be inserted into the subject (8) and depicted in the X-Ray image.

13. A computer program product comprising instructions which, when the program is executed by a computer, cause the computer to carry out and/or control the steps of the method of any of claims 1 to 11.

14. A computer-readable medium comprising instructions which, when executed by a computer, cause the computer to carry out and/or control the steps of the method of any of claims 1 to 11.
